(19) Europäisches Patentamt
European Patent Office
Office européen des brevets

(11) Veröffentlichungsnummer: **0 110 225**
**B1**

(12) # EUROPÄISCHE PATENTSCHRIFT

(45) Veröffentlichungstag der Patentschrift: **23.10.85**

(21) Anmeldenummer: **83111353.5**

(22) Anmeldetag: **14.11.83**

(51) Int. Cl.⁴: **C 07 C 153/07,** C 07 D 207/16, C 07 D 209/26, C 07 D 205/04, C 07 D 211/60, C 07 D 217/26, C 07 D 333/24, C 07 D 277/06

(54) **Bis-(benzoylthio)-carbonsäuren, ihre Herstellung und Verwendung zur Herstellung von Wirkstoffen.**

(30) Priorität: **24.11.82 DE 3243369**

(43) Veröffentlichungstag der Anmeldung: **13.06.84 Patentblatt 84/24**

(45) Bekanntmachung des Hinweises auf die Patenterteilung: **23.10.85 Patentblatt 85/43**

(84) Benannte Vertragsstaaten: **AT BE CH DE FR GB IT LI NL SE**

(56) Entgegenhaltungen:
**EP - A - 0 018 104**
**DE - A - 2 752 720**
**DE - A - 3 012 140**
**DE - A - 3 212 766**
**GB - A - 758 897**

**HOUBEN-WEYL "Methoden der organischen Chemie", 4. Auflage, Band IX: "Schwefel-, Selen-, Tellurverbindungen", 1955 GEORG THIEME VERLAG, Stuttgart Seiten 749-756**

(73) Patentinhaber: **BASF Aktiengesellschaft, Carl-Bosch-Strasse 38, D-6700 Ludwigshafen (DE)**

(72) Erfinder: **Kubinyi, Hugo, Dr., Donnersbergstrasse 9, D-6714 Weisenheim/Sand (DE)**
Erfinder: **Traut, Martin, Dr., Muehltalstrasse 125, D-6900 Heidelberg (DE)**
Erfinder: **Gries, Josef, Dr., Roemerweg 43, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 110 225 B1

BUNDESDRUCKEREI BERLIN

**Beschreibung**

Die Erfindung betrifft neue Bis-(benzoylthio)-carbonsäuren, Verfahren zu deren Herstellung sowie deren Verwendung zur Herstellung von Wirkstoffen.

Einfache Acetylthio- und Benzoylthioverbindungen sind seit langer Zeit bekannt. Man stellt sie entweder durch Addition der Thiosäuren an ungesättigte Verbindungen, durch Alkylierung der Thiosäuren mit Alkylhalogeniden oder durch Acylierung von Mercaptanen dar (s. Houben—Weyl, Methoden der organischen Chemie, Bd. IX, S. 749 bis 756). Von Propionsäure und Isobuttersäure sind auch Bis-(acetylthio)-derivate bekannt (DE-OS 2 752 720). Dagegen sind Bis-(benzoylthio)-derivate einfacher aliphatischer Carbonsäuren bisher noch nicht beschrieben worden. Es wurde nun gefunden, daß solche Bis-(benzoylthio)-carbonsäuren nicht nur chemisch einfach zugänglich sind, sondern darüber hinaus auch wertvolle Zwischenprodukte für die Synthese neuer Arzneimittel-Wirkstoffe darstellen.

Gegenstand der Erfindung sind Bis-(benzoylthio)-carbonsäuren der Formel I

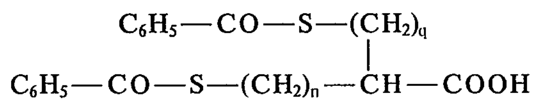

(I)

in der
n und q gleich oder verschieden sind und die Zahlen 0 oder 1 bedeuten.

Die neuen Verbindungen werden hergestellt, indem man

a) Verbindungen der Formel II

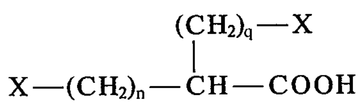

(II)

worin n und q die angegebene Bedeutung haben und X ein Halogenatom darstellt, mit Thiobenzoesäure oder

b) — falls n und q beide die Zahl 1 bedeuten — eine Verbindung der Formel III

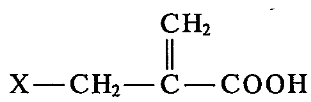

(III)

in der R und X die angegebene Bedeutung haben, mit Thiobenzoesäure umsetzt

und die so erhaltenen Verbindungen — falls $n \neq q$ — gegebenenfalls in ihre optischen Antipoden trennt.

Die Reaktionen a) und b) gelingen gut in einem organischen Lösungsmittel, vorzugsweise Toluol, Tetrahydrofuran, Dioxan, Ethylenglykoldimethylether, Acetonitril oder Dimethylformamid, bzw. einem organisch-wäßrigen Medium, vorzugsweise Tetrahydrofuran/Wasser oder Dioxan/Wasser, in Gegenwart einer anorganischen Base, vorzugsweise Kaliumcarbonat, bei Temperaturen zwischen 0 und 40°C.

Als Halogenderivate verwendet man vorzugsweise die Chlor-, Brom- oder Iod-Derivate.

Die Verbindungen I liegen — falls $n \neq q$ — als Racemate vor. Diese können in üblicher Weise in ihre Antipoden getrennt werden. Das kann mit Hilfe einer optisch aktiven Base, z. B. (+)- oder (—)-Ephedrin geschehen.

Die neuen Verbindungen eignen sich zur Herstellung von Verbindungen der Formel IV

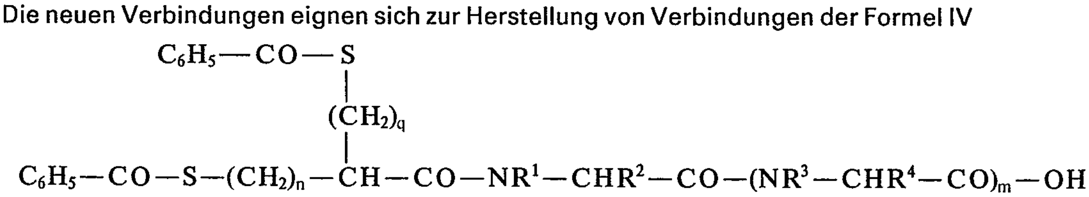

(IV)

worin
$R^1$ und $R^3$ gleich oder verschieden sind und Wasserstoffatome oder $C_{1-4}$-Alkylgruppen darstellen,
$R^2$ und $R^4$ gleich oder verschieden sind und Wasserstoff, einen gegebenenfalls durch eine Hydroxy-, Mercapto-, $C_{1-4}$-Alkylthio-, Carbonsäureester-, Carbonamido- oder Acylaminogruppe substituierten $C_{1-6}$-Alkylrest oder einen gegebenenfalls im Arylrest durch Hydroxy-, $C_{1-4}$-Alkoxy-, Arylalkoxygruppen oder Halogenatome substituierten Aryl-, Heteroaryl-, Arylalkylen- oder Heteroarylalkylenrest bedeuten, wobei $R^1$ und $R^2$ und/oder $R^3$ und $R^4$ zusammen auch die Reste $-(CH_2)_p-$ ($p = 2, 3, 4$), $-CH_2-S-CH_2-$ oder $-CH_2-C_6H_4-CH_2-$ (ortho) sein können und
q, m und n gleich oder verschieden sind und die Zahlen 0 oder 1 bedeuten,

sowie deren Salze mit physiologisch verträglichen Basen.

Hierzu werden die Verbindungen I nach in der Peptidchemie üblichen Verfahren mit Verbindungen der Formel V

$$R^1NH-CHR^2-CO-(NR^3-CHR^4-CO)_m-OH \qquad (V)$$

worin $R^1$, $R^2$, $R^3$, $R^4$ und m die angegebene Bedeutung haben, umgesetzt.

Die Verbindungen IV eignen sich als Arzneimittelwirkstoffe zur Behandlung von Hypertonie und Schmerzzuständen.

Die folgenden Beispiele sollen die Erfindung erläutern.

Alle Schmelzpunkte sind unkorrigiert. Angaben zur Dünnschichtchromatographie beziehen sich auf die Systeme

I = Essigsäureethylester/Eisessig = 20/1
II = Cyclohexan/Essigsäureethylester/Eisessig = 10/10/1.

Die Dünnschichtchromatographie wurde an Kieselgel 60 $F_{254}$-Platten der Firma Merck, Darmstadt, unter Kammersättigung durchgeführt. Die Laufstrecke betrug ca. 13 cm.

## a) Herstellung von Verbindungen der Formel I

### Beispiel 1

### 2,2-Bis-(benzoylthio)-essigsäure

Zu einer gut gerührten Suspension von 110,6 g (0,8 Mol) wasserfreiem Kaliumcarbonat in 150 ml trockenem Acetonitril tropfte man bei 0 bis 5°C eine Lösung von 43,6 g (0,2 Mol) Dibromessigsäure und 60,8 g (0,44 Mol) Thiobenzoesäure in 100 ml trockenem Acetonitril. Man ließ noch 45 min bei 15 bis 20°C nachrühren, trug den Ansatz anschließend in 1 l Eiswasser ein und extrahierte zweimal mit je 400 ml Diethylether. Die vereinigten Etherphasen wurden noch einmal mit 500 ml 10%iger wäßriger Kaliumcarbonatlösung extrahiert und die vereinigten wäßrigen Phasen unter Kühlung mit 2N wäßriger $H_2SO_4$ auf pH 3 gebracht. Man extrahierte zweimal mit je 400 ml Essigsäureethylester, trocknete die organischen Phasen mit wasserfreiem Natriumsulfat und dampfte ein. Das zurückbleibende Öl wurde aus Benzol kristallisiert. Nach Absaugen und Waschen mit Benzol/Petrolether erhielt man 48,4 g (59%) 2,2-Bis-(benzoylthio)-essigsäure als Komplex mit 1 Mol Kristallbenzol, Fp. 82 bis 83°C; $R_f = 0,52$ in System I.

Analog erhielt man 3,3'-Bis-(benzoylthio)-isobuttersäure, vgl. Beispiel 3.

### Beispiel 2

### 2,3-Bis-(benzoylthio)-propionsäure

Zu einer auf 0°C gekühlten, gut gerührten Suspension von 276,4 g (2,0 Mol) wasserfreiem, gepulvertem Kaliumcarbonat in 700 ml Toluol wurden 115,9 g (0,5 Mol) 2,3-Dibrompropionsäure und 138,2 g (1,0 Mol) Thiobenzoesäure gegeben. Man rührte noch kurze Zeit bei 0°C und ließ dann die Temperatur des Reaktionsgemisches langsam ansteigen. Bei etwa 20°C trat spontan exotherme Umsetzung ein; durch externe Kühlung mit Eiswasser wurde die Reaktionstemperatur unter 40°C gehalten. Nach Ende der Reaktion wurde mit 1 l Toluol verdünnt, abdekantiert, der Rückstand noch einmal mit Toluol digeriert und in 1 l Eiswasser eingetragen. Nach Ansäuern mit 2N wäßriger $H_2SO_4$ wurde das Reaktionsprodukt zweimal mit je 500 ml Essigsäureethylester extrahiert. Die organischen Phasen wurden mit wasserfreiem Natriumsulfat getrocknet und anschließend unter vermindertem Druck eingedampft. Nach Kristallisation aus Tetrachlorkohlenstoff/Cyclohexan erhielt man 117,8 g (68%) 2,3-Bis-(benzoylthio)-propionsäure, Fp. 116 bis 118°C, $R_f = 0,41$ in System II.

200 g der so erhaltenen racemischen 2,3-Bis-(benzoylthio)-propionsäure wurden in 1,5 l Isopropanol gelöst und mit einer Lösung von 106 g (−)-Ephedrin in 500 ml Isopropanol versetzt. Die resultierenden Kristalle wurden aus Methylenchlorid/Isopropanol umkristallisiert. Man erhielt 135,2 g Salz. Fp. 127 bis 129°C, $[\alpha]_D^{20} = -28,9°$ (c = 1 in Chloroform). Nach Freisetzung der Säure durch Ausschütteln zwischen Essigsäureethylester und 10%iger wäßriger Kaliumhydrogensulfat-Lösung und Kristallisation des Eindampfrückstandes der organischen Phase aus Diethylether/Petrolether erhielt man zuerst ein Gemisch aus Racemat und (R)-Form und anschließend aus der Mutterlauge 33,7 g (R)-2,3-Bis-(benzoylthio)-propionsäure, Fp. 95 bis 96°C, $[\alpha]_D^{20} = -99°$ (c = 1 in Chloroform). Aus der Mutterlauge der Kristallisation des (−)-Ephedrin-Salzes wurde die Säure in Freiheit gesetzt und ein Salz mit (+)-Ephedrin gebildet. Man erhielt 97,8 g Salz, Fp. 127 bis 129°C, $[\alpha]_D^{20} = +33,9°$ (c = 1 in Chloroform). Spaltung

des Salzes und Kristallisation aus Diethylether/Petrolether lieferte zuerst ein Gemisch aus Racemat und (S)-Form und anschließend aus der Mutterlauge 26,6 g (S)-2,3-Bis-(benzoylthio)-propionsäure, Fp. 95 bis 96°C, $[\alpha]_D^{20} = +103°$ (c = 1 in Chloroform). Neben den beiden Antipoden wurden insgesamt 122,4 g racemische 2,3-Bis-(benzoylthio)-propionsäure wiedergewonnen, die erneut für eine Racematspaltung eingesetzt werden können.

Beispiel 3

3,3'-Bis-(benzoylthio)-isobuttersäure

Zu einer Lösung von 10,6 g (50 mMol) 2-Iodmethyl-acrylsäure und 13,8 g (100 mMol) Kaliumcarbonat in 100 ml Dioxan/Wasser = 2/3 wurden unter Rühren 14,5 g (105 mMol) Thiobenzoesäure zugetropft. Nach 3 h Rühren bei Raumtemperatur wurde der Ansatz mit 500 ml Wasser verdünnt, mit 1N wäßriger Schwefelsäure auf pH 3 gebracht und zweimal mit je 300 ml Essigsäureethylester ausgeschüttelt. Die vereinigten organischen Phasen wurden noch einmal mit wenig Wasser gewaschen, mit wasserfreiem Natriumsulfat getrocknet und unter vermindertem Druck eingedampft. Nach Kristallisation des Rückstandes aus Cyclohexan erhielt man 15,5 g (86%) 3,3'-Bis-(benzoylthio)-isobuttersäure, Fp. 107 bis 109°C, $R_f = 0{,}45$ in System II.

b) Verwendung der gemäß Beispiel 1 bis 3 erhaltenen Verbindungen

Beispiel A

16,4 g (40 mMol) 2,2-Bis-(benzoylthio)-essigsäure (Beispiel 1) wurden in 150 ml Toluol suspendiert und unter Kühlung mit 25,4 g (0,2 Mol) Oxalsäuredichlorid versetzt. Man erwärmte 2 h auf 50°C, dampfte anschließend unter vermindertem Druck ein und entfernte überschüssiges Oxalsäuredichlorid durch mehrmaliges Koevaporieren mit Toluol.

14,2 g (40 mMol) so erhaltenes rohes 2,2-Bis-(benzoylthio)-acetylchlorid wurden in 100 ml trockenem Dioxan gelöst und zu einer Suspension von 3,6 g (40 mMol) Alanin in 100 ml trockenem Dioxan gegeben. Das Reaktionsgemisch wurde 2 h unter Rückfluß gekocht und anschließend das Lösungsmittel unter vermindertem Druck abgezogen. Das ölige Rohprodukt wurde an desaktiviertem Kieselgel mit Cyclohexan/Essigsäureethylester = 2/1 säulenchromatographiert. Man erhielt 10,3 g (64%) N-[2,2-Bis-(benzoylthio)-acetyl]-alanin, Fp. 161 bis 162°C (Benzol), $R_f = 0{,}56$ in System I.

Analog wurden erhalten:

N-[2,2-Bis-(benzoylthio)-acetyl]-glycin, Fp. 141 bis 143°C (Diisopropylether), $R_f = 0{,}50$ in System I.

N-[2,2-Bis-(benzoylthio)-acetyl]-leucin, Fp. 104 bis 105°C (Tetrachlorkohlenstoff), $R_f = 0{,}63$ in System I.

N-[2,2-Bis-(benzoylthio)-acetyl]-methionin, Fp. 121 bis 122°C (Benzol/Cyclohexan), $R_f = 0{,}58$ in System I.

N-[2,2-Bis-(benzoylthio)-acetyl]-prolin, Fp. 80 bis 87°C (nach Fällung aus Diethylether/Petrolether), $R_f = 0{,}49$ in System I.

N-[2,2-Bis-(benzoylthio)-acetyl]-phenylalanin, Fp. 161 bis 162°C (Benzol), $R_f = 0{,}59$ in System I.

N-[2,2-Bis-(benzoylthio)-acetyl]-p-chlorphenylalanin, Fp. 141 bis 142°C (Benzol), $R_f = 0{,}58$ in System I.

D,L-2-[2,2-Bis-(benzoylthio)-acetamido]-4-phenylbuttersäure, Fp. 157 bis 158°C (Benzol), $R_f = 0{,}60$ in System I.

N-[2,2-Bis-(benzoylthio)-acetyl]-tryptophan, Fp. ab 80°C (Diethylether/Petrolether), $R_f = 0{,}58$ in System I.

N-[2,2-Bis-(benzoylthio)-acetyl]-alanyl-prolin, Fp. 199 bis 200°C (Essigsäureethylester), $R_f = 0{,}32$ in System I.

Beispiel B

28,8 g (80 mMol) 2,3-Bis-(benzoylthio)-propionsäure (Beispiel 2) wurden in 300 ml Toluol suspendiert und unter Kühlung mit 50,8 g (0,4 Mol) Oxalsäuredichlorid versetzt.

Man erwärmte 2 h auf 50°C, dampfte anschließend unter vermindertem Druck ein und entfernte überschüssiges Oxalsäuredichlorid durch mehrmaliges Koevaporieren mit Toluol. Das verbleibende rohe 2,3-Bis-(benzoylthio)-propionylchlorid kann direkt für weitere Umsetzungen verwendet werden.

18,4 g (50 mMol) des so erhaltenen Rohprodukts wurden in 100 ml trockenem Dioxan gelöst und zu einer Suspension von 3,8 g (50 mMol) Glycin in 100 ml trockenem Dioxan gegeben. Das Reaktionsgemisch wurde 2 h unter Rückfluß gekocht und anschließend das Lösungsmittel unter vermindertem Druck abgezogen. Kristallisation des Rückstandes aus Essigsäureethylester lieferte 9,3 g (46%) N-[2,3-Bis-(benzoylthio)-propionyl]-glycin, Fp. 160 bis 163° C, $R_f$ = 0,59 in System I. Aus der Mutterlauge erhielt man nach Säulenchromatographie an desaktiviertem Kieselgel mit dem System Cyclohexan/Essigsäureethylester = 1/1 weiteres dc-reines Produkt, Fp. 162 bis 164° C.

Analog wurden erhalten (die Reinigung der Rohprodukte erfolgte durch Säulenchromatographie mit den Systemen Cyclohexan/Essigsäureethylester = 3/1 bis 1/1):

N-[2,3-Bis-(benzoylthio)-propionyl]-sarcosin, $R_f$ = 0,58 in System I.

N-[2,3-Bis-(benzoylthio)-propionyl]-alanin, Fp. 152 bis 155° C (Cyclohexan/Essigsäureethylester), $R_f$ = 0,32 in System II.

N-[2,3-Bis-(benzoylthio)-propionyl]-D,L-norleucin als Diastereomerengemisch, Fp. 126 bis 127° C (Diethylether), $R_f$ = 0,39 in System II.

N-[2,3-Bis-(benzoylthio)-propionyl]-leucin als Diastereomerengemisch, Fp. 90 bis 93° C (Cyclohexan/Essigsäureethylester), $R_f$ = 0,38 in System II.

N-[2,3-Bis-(benzoylthio)-propionyl]-threonin als Diastereomerengemisch, Fp. 156 bis 159° C (Diethylether), $R_f$ = 0,44 und 0,40 in System I.

N-[2,3-Bis-(benzoylthio)-propionyl]-cystein als Diastereomerengemisch, Fp. 171 bis 173° C (Essigsäureethylester), $R_f$ = 0,34 in System II.

N-[2,3-Bis-(benzoylthio)-propionyl]-L-penicillamin als Diastereomerengemisch, Fp. 114 bis 117° C (Diisopropylether), $R_f$ = 0,43 in System II.

N-[2,3-Bis-(benzoylthio)-propionyl]-methionin als Diastereomerengemisch, Fp. 124 bis 127° C (Cyclohexan/Essigsäureethylester); $R_f$ = 0,38 in System II.

N-[2,3-Bis-(benzoylthio)-propionyl]-glutaminsäure-$\gamma$-benzylester als Diastereomerengemisch, Fp. 93 bis 96° C (Diethylether/Petrolether), $R_f$ = 0,36 in System II.

$N_\alpha$-[2,3-Bis-(benzoylthio)-propionyl]-glutamin als Diastereomerengemisch, $R_f$ = 0,48 in System I.

$N_\alpha$-[2,3-Bis-(benzoylthio)-propionyl]-$N_\varepsilon$-(benzyloxycarbonyl)-lysin als Diastereomerengemisch, Fp. 107 bis 110° C (Diethylether), $R_f$ = 0,64 in System I.

N-[2,3-Bis-(benzoylthio)-propionyl]-D,L-azetidin-2-carbonsäure als Diastereomerengemisch, $R_f$ = 0,41 und 0,37 in System I.

N-[2,3-Bis-(benzoylthio)-propionyl]-D,L-piperidin-2-carbonsäure als Diastereomerengemisch, Fp. 162 bis 165° C, $R_f$ = 0,66 in System I.

N-[2,3-Bis-(benzoylthio)-propionyl]-D,L-1,2,3,4-tetrahydroisochinolin-3-carbonsäure als Diastereomerengemisch, $R_f$ = 0,67 in System I.

N-[2,3-Bis-(benzoylthio)-propionyl]-D,L-$\alpha$-phenylglycin als Diastereomerengemisch, Fp. 169 bis 179° C (Diethylether), $R_f$ = 0,37 in System II.

N-[2,3-Bis-(benzoylthio)-propionyl]-D,L-$\alpha$-(thien-3-yl)-glycin als Diastereomerengemisch, Fp. 161 bis 164° C (Diethylether), $R_f$ = 0,35 in System II.

N-[2,3-Bis-(benzoylthio)-propionyl]-phenylalanin als Diastereomerengemisch, Fp. 104 bis 107° C (Toluol/Petrolether), $R_f$ = 0,39 in System II.

N-[2,3-Bis-(benzoylthio)-propionyl]-tyrosin als Diastereomerengemisch, $R_f$ = 0,27 in System II.

N-[2,3-Bis-(benzoylthio)-propionyl]-D,L-(3,4-dimethoxy)-phenylalanin als Diastereomerengemisch, $R_f$ = 0,25 in System II.

N-[2,3-Bis-(benzoylthio)-propionyl]-O-benzyl-tyrosin als Diastereomerengemisch, Fp. 173 bis 176° C (Essigsäureethylester/Petrolether), $R_f$ = 0,38 in System II.

N-[2,3-Bis-(benzoylthio)-propionyl]-p-chlorphenylalanin als Diastereomerengemisch, $R_f$ = 0,37 in System II.

N-[2,3-Bis-(benzoylthio)-propionyl]-D,L-3-(naphth-1-yl)-alanin als Diastereomerengemisch, $R_f$ = 0,37 in System II.

N-[2,3-Bis-(benzoylthio)-propionyl]-D,L-3-(naphth-2-yl)-alanin als Diastereomerengemisch, Fp. 104 bis 143° C (Cyclohexan/Essigsäureethylester), $R_f$ = 0,36 in System II.

D,L-2-[2,3-Bis-(benzoylthio)-propionamido]-4-phenylbuttersäure als Diastereomerengemisch, Fp. 157 bis 160° C (Essigsäureethylester/Petrolether), $R_f$ = 0,38 in System II.

N-[2,3-Bis-(benzoylthio)-propionyl]-tryptophan als Diastereomerengemisch, Fp. 180 bis 183° C (Essigsäureethylester), $R_f$ = 0,34 in System II.

N-[2,3-Bis-(benzoylthio)-propionyl]-D-tryptophan als Diastereomerengemisch, Fp. 182 bis 185° C (Essigsäureethylester), $R_f$ = 0,34 in System II.

N-[2,3-Bis-(benzoylthio)-propionyl]-N-methyl-tryptophan als Diastereomerengemisch, $R_f$ = 0,35 in System II.

N-[2,3-Bis-(benzoylthio)-propionyl]-prolyl-prolin als Diastereomerengemisch, $R_f$ = 0,22 in System I.

N-[2,3-Bis-(benzoylthio)-propionyl]-phenylalanyl-alanin als Diastereomerengemisch, Fp. 110 bis 113° C (Diethylether), $R_f$ = 0,62 in System I.

N-[2,3-Bis-(benzoylthio)-propionyl]-tryptophyl-prolin als Diastereomerengemisch, $R_f$ = 0,62 in System I.

## Beispiel C

Man verfuhr wie in Beispiel B und verwendete Serin statt Glycin. Nach Säulenchromatographie an desaktiviertem Kieselgel mit dem System Cyclohexan/Essigsäureethylester = 1/1 erhielt man N-[2,3-Bis-(benzoylthio)-propionyl]-serin als Diastereomerengemisch. Fraktionierte Kristallisation aus Essigsäureethylester lieferte die Diastereomeren N-[(R)-2,3-Bis-(benzoylthio)-propionyl]-serin, Fp. 184 bis 187°C, $R_f$ = 0,35 in System I, $[\alpha]_D^{20}$ = −60° (c = 1 in Chloroform/Methanol = 9/1) und N-[(S)-2,3-Bis-(benzoylthio)-propionyl]-serin, Fp. 147 bis 150°C, $R_f$ = 0,39 in System I, $[\alpha]_D^{20}$ = +116° (c = 1 in Chloroform/Methanol = 9/1).

## Beispiel D

Man verfuhr wie in Beispiel B und verwendete Valin statt Glycin. Nach Säulenchromatographie an desaktiviertem Kieselgel mit dem System Cyclohexan/Essigsäureethylester = 3/1 erhielt man N-[2,3-Bis-(benzoylthio)-propionyl]-valin als Diastereomerengemisch. Fraktionierte Kristallisation aus Diethylether und Diethylether/Petrolether liefert die Diastereomeren N-[(S)-2,3-Bis-(benzoylthio)-propionyl]-valin, Fp. 157 bis 160°C, $R_f$ = 0,39 in System II, $[\alpha]_D^{20}$ = +152° (c = 1 in Chloroform), und N-[(R)-2,3-Bis-(benzoylthio)-propionyl]-valin, Fp. 83 bis 90°C, $R_f$ = 0,37 in System II, $[\alpha]_D^{20}$ = −69° (c = 1 in Chloroform).

## Beispiel E

Man verfuhr wie in Beispiel B und verwendete Isoleucin statt Glycin. Nach Säulenchromatographie an desaktiviertem Kieselgel mit dem System Cyclohexan/Essigsäureethylester = 3/1 erhielt man N-[2,3-Bis-(benzoylthio)-propionyl]-isoleucin als Diastereomerengemisch. Fraktionierte Kristallisation aus Diethylether und Diisopropylether lieferte die Diastereomeren N-[(S)-2,3-Bis-(benzoylthio)-propionyl]-isoleucin, Fp. 151 bis 153°C, $R_f$ = 0,39 in System II, $[\alpha]_D^{20}$ = +135° (c = 1 in Chloroform), und N-[(R)-2,3-Bis-(benzoylthio)propionyl]-isoleucin, Fp. 94 bis 95°C, $R_f$ = 0,39 in System II, $[\alpha]_D^{20}$ = −65° (c = 1 in Chloroform).

## Beispiel F

Man verfuhr wie in Beispiel B und verwendete L-Thiazolidin-4-carbonsäure statt Glycin. Nach Säulenchromatographie an desaktiviertem Kieselgel mit dem System Cyclohexan/Essigsäureethylester = 2/1 erhielt man die beiden Diastereomeren N-[(S)-2,3-Bis-(benzoylthio)-propionyl]-L-thiazolidin-4-carbonsäure, Fp. 133 bis 138°C (Diethylether), $R_f$ = 0,61 in System I, $[\alpha]_D^{20}$ = +82° (c = 1 in Chloroform), und N-[(R)-2,3-Bis-(benzoylthio)-propionyl]-L-thiazolidin-4-carbonsäure, $R_f$ = 0,57 in System I, $[\alpha]_D^{20}$ = −145° (c = 1 in Chloroform).

Analog wurden erhalten:

N-[(R)-2,3-Bis-(benzoylthio)-propionyl]-prolylphenylalanin, $R_f$ = 0,50 in System I, $[\alpha]_D^{20}$ = −133° (c = 1 in Chloroform).

N-[(S)-2,3-Bis-(benzoylthio)-propionyl]-prolyl-phenylalanin, $R_f$ = 0,45 in System I, $[\alpha]_D^{20}$ = +41° (c = 1 in Chloroform).

## Beispiel G

Man verfuhr wie in Beispiel B und verwendete Alanyl-prolin statt Glycin. Das Umsetzungsprodukt kristallisierte direkt aus dem Reaktionsansatz. Umkristallisation aus Isopropanol lieferte N-[2,3-Bis-(benzoylthio)-propionyl]-alanyl-prolin als Diastereomerengemisch, Fp. 185 bis 186°C, $R_f$ = 0,40 in System I, $[\alpha]_D^{20}$ = −39° (c = 1 in Chloroform). Fraktionierte Kristallisation des Gemisches aus Essigsäureethylester und Essigsäureethylester/Diethylether lieferte die Diastereomeren: N-[(R)-2,3-Bis-(benzoylthio)-propionyl]-alanyl-prolin, Fp. 196 bis 197°C, $R_f$ = 0,40 in System I, $[\alpha]_D^{20}$ = −122° (c = 1 in Chloroform), und N-[(S)-2,3-Bis-(benzoylthio)-propionyl]-alanylprolin, Fp. 172 bis 173°C, $R_f$ = 0,40 in in System I, $[\alpha]_D^{20}$ = +36° (c = 1 in Chloroform).

## Beispiel H

4,4 g (25 mMol) Prolin-tert.-butylester (Rohprodukt, erhalten durch Hydrierung von 7,6 g Z-Prolin-tert.-butylester) in 100 ml Tetrahydrofuran wurden unter Kühlung auf 0° C mit einer Lösung von 8,7 g (25 mMol) (S)-2,3-Bis-(benzoylthio)-propionsäure und 4,8 g (25 mMol) 1-Ethyl-3-(3-dimethylaminopropyl)-carbodiimid-hydrochlorid in 125 ml Tetrahydrofuran/Wasser = 4/1 versetzt. Nach 1 h Rühren bei 0° C wurde noch 1 h bei 20° C nachgerührt, der Ansatz zwischen Essigsäureethylester und Wasser ausgeschüttelt und die organische Phase nach Trocknen mit wasserfreiem Natriumsulfat unter vermindertem Druck eingedampft. Säulenchromatographie an desaktiviertem Kieselgel mit dem System Cyclohexan/Essigsäureethylester = 2/1 lieferte 9,0 g (72%) N-[(S)-2,3-Bis-(benzoylthio)-propionyl]-prolin-tert.-butylester. Das Rohprodukt wurde in 50 ml trockenem Dioxan gelöst und mit 50 ml 6N HCl/Dioxan 1 h bei 20° C stehengelassen. Nach Ausschüttelung zwischen Essigsäureethylester und Wasser, zweimaligem Waschen der organischen Phase mit Wasser, Trocknen mit wasserfreiem Natriumsulfat und Eindampfen unter vermindertem Druck erhielt man 7,8 g (70%) N-[(S)-2,3-Bis-(benzoylthio)-propionyl]-prolin, Fp. 155 bis 158° C (Cyclohexan/Essigsäureethylester), $R_f$ = 0,56 in System I, $[\alpha]_D^{20} = +76°$ (c = 1 in Chloroform).

Analog Beispiel H wurden erhalten (die Reinigung der Rohprodukte erfolgte durch Säulenchromatographie an desaktiviertem Kieselgel mit den Systemen Cyclohexan/Essigsäureethylester = 3 : 1 bis 1 : 1):

N-[(R)-2,3-Bis-(benzoylthio)-propionyl]-prolin, $R_f$ = 0,54 in System I, $[\alpha]_D^{20} = -117°$ (c = 1 in Chloroform).

N-[(S)-2,3-Bis-(benzoylthio)-propionyl]-tryptophan, $R_f$ = 0,34 in System II, $[\alpha]_D^{20} = +84°$ (c = 1 in Chloroform).

N-[(R)-2,3-Bis-(benzoylthio)-propionyl]-tryptophan, $R_f$ = 0,34 in System II, $[\alpha]_D^{20} = -77°$ (c = 1 in Chloroform).

N-[(S)-2,3-Bis-(benzoylthio)-propionyl]-alanyl-prolin, Fp. 175 bis 176° C (Essigsäureethylester), $R_f$ = 0,40 in System I, $[\alpha]_D^{20} = +36°$ (c = 1 in Chloroform).

N-[(R)-2,3-Bis-(benzoylthio)-propionyl]-alanyl-prolin, Fp. 195 bis 196° C (Essigsäureethylester), $R_f$ = 0,40 in System I, $[\alpha]_D^{20} = -125°$ (c = 1 in Chloroform).

## Beispiel I

Man verfuhr wie in Beispiel H und verwendete N-[(S)-2,3-Bis-(benzoylthio)-propionyl]-valin statt (S)-2,3-Bis-(benzoylthio)-propionsäure. Nach Säulenchromatographie an desaktiviertem Kieselgel mit dem System Cyclohexan/Essigsäureethylester = 1/1 erhielt man N-[(S)-2,3-Bis-(benzoylthio)-propionyl]-valyl-prolin, Fp. 85 bis 93° C (Diethylether/Pentan), $R_f$ = 0,54 in System I, $[\alpha]_D^{20} = +53°$ (c = 1 in Chloroform).

Analog Beispiel I wurden hergestellt:

N-[(R)-2,3-Bis-(benzoylthio)-propionyl]-valyl-prolin, Fp. 97 bis 100° C (Diethylether), $R_f$ = 0,54 in System I, $[\alpha]_D^{20} = -114°$ (c = 1 in Chloroform).

N-[(S)-2,3-Bis-(benzoylthio)-propionyl]-valyl-tryptophan, Fp. 173 bis 181° C (Toluol), $R_f$ = 0,60 in System I.

N-[(R)-2,3-Bis-(benzoylthio)-propionyl]-valyl-tryptophan, Fp. 205 bis 208° C (Toluol), $R_f$ = 0,60 in System I.

## Beispiel J

14,4 g (40 mMol) 3,3'-Bis-(benzoylthio)-isobuttersäure (Beispiel 3) wurden in 120 ml Toluol suspendiert und unter Kühlung mit 25,4 g (200 mMol) Oxalsäuredichlorid versetzt. Man erwärmte 2 h auf 50° C, dampfte anschließend unter vermindertem Druck ein und entfernte überschüssiges Oxalsäuredichlorid durch mehrmaliges Koevaporieren mit Toluol.

Der Rückstand wurde in 150 ml trockenem Dioxan aufgenommen und zusammen mit 3,0 g (40 mMol) Glycin 2 h unter Rückfluß gekocht. Anschließend dampfte man unter vermindertem Druck ein. Säulenchromatographie an desaktiviertem Kieselgel mit dem System Cyclohexan/Essigsäureethylester = 2/1 lieferte 12,7 g (76%) N-[3,3'-Bis-(benzoylthio)-isobutyryl]-glycin, Fp. 113 bis 114° C (Diethylether), $R_f$ = 0,58 in System I.

Analog erhielt man (die Reinigung der Rohprodukte erfolgte durch Säulenchromatographie an desaktiviertem Kieselgel mit den Systemen Cyclohexan/Essigsäureethylester = 3/1 bis 1/1):

N-[3,3'-Bis-(benzoylthio)-isobutyryl]-sarcosin, Fp. 130 bis 132°C (Diethylether/Petrolether), $R_f$ = 0,59 in System I.
N-[3,3'-Bis-(benzoylthio)-isobutyryl]-alanin, Fp. 145 bis 146°C (Diethylether), $R_f$ = 0,31 in System II.
N-[3,3'-Bis-(benzoylthio)-isobutyryl]-D-alanin, Fp. 144 bis 145°C (Diethylether), $R_f$ = 0,31 in System II.
N-[3,3'-Bis-(benzoylthio)-isobutyryl]-D,L-norleucin, Fp. 132 bis 133°C (Diethylether/Petrolether), $R_f$ = 0,39 in System II.
N-[3,3'-Bis-(benzoylthio)-isobutyryl]-valin, $R_f$ = 0,38 in System II.
N-[3,3'-Bis-(benzoylthio)-isobutyryl]-isoleucin, $R_f$ = 0,38 in System II.
N-[3,3'-Bis-(benzoylthio)-isobutyryl]-leucin, Fp. 93 bis 94°C (Diethylether/Petrolether), $R_f$ = 0,37 in System II.
N-[3,3'-Bis-(benzoylthio)-isobutyryl]-methionin, Fp. 113 bis 114°C (Diethylether/Petrolether), $R_f$ = 0,36 in System II.
N-[3,3'-Bis-(benzoylthio)-isobutyryl]-serin, $R_f$ = 0,38 in System I.
N-[3,3'-Bis-(benzoylthio)-isobutyryl]-cystein, $R_f$ = 0,35 in System II.
N-[3,3'-Bis-(benzoylthio)-isobutyryl]-prolin, Fp. 137 bis 138°C (Essigsäureethylester), $R_f$ = 0,56 in System I.
N-[3,3'-Bis-(benzoylthio)-isobutyryl]-D-prolin, Fp. 136 bis 138°C (Essigsäureethylester), $R_f$ = 0,56 in System I.
N-[3,3'-Bis-(benzoylthio)-isobutyryl]-thiazolidin-4-carbonsäure, Fp. 127 bis 129°C (Diethylether), $R_f$ = 0,60 in System I.
N-[3,3'-Bis-(benzoylthio)-isobutyryl]-D,L-azetidin-2-carbonsäure, $R_f$ = 0,43 in System I.
N-[3,3'-Bis-(benzoylthio)-isobutyryl]-D,L-piperidin-2-carbonsäure, $R_f$ = 0,67 in System I.
N-[3,3'-Bis-(benzoylthio)-isobutyryl]-D,L-1,2,3,4-tetrahydroisochinolin-3-carbonsäure, $R_f$ = 0,66 in System I.
N-[3,3'-Bis-(benzoylthio)-isobutyryl]-D,L-phenyl-glycin, Fp. 158 bis 160°C (Diethylether/Petrolether), $R_f$ = 0,37 in System II.
N-[3,3'-Bis-(benzoylthio)-isobutyryl]-phenylalanin, Fp. 57 bis 60°C (Diethylether/Petrolether), $R_f$ = 0,39 in System II.
N-[3,3'-Bis-(benzoylthio)-isobutyryl]-D-phenylalanin, Fp. 59 bis 61°C (Diethylether/Petrolether), $R_f$ = 0,39 in System II.
N-[3,3'-Bis-(benzoylthio)-isobutyryl]-tyrosin, Fp. 71 bis 73°C (Petrolether), $R_f$ = 0,27 in System II.
N-[3,3'-Bis-(benzoylthio)-isobutyryl]-D,L-(3,4-dimethoxy)-phenylalanin, Fp. 159 bis 161°C (Essigsäureethylester), $R_f$ = 0,25 in System II.
N-[3,3'-Bis-(benzoylthio)-isobutyryl]-O-benzyl-tyrosin, $R_f$ = 0,36 in System II.
N-[3,3'-Bis-(benzoylthio)-isobutyryl]-p-chlorphenylalanin, Fp. 143 bis 146°C (Diethylether), $R_f$ = 0,35 in System II.
N-[3,3'-Bis-(benzoylthio)-isobutyryl]-D,L-3-(naphth-1-yl)-alanin, $R_f$ = 0,37 in System II.
N-[3,3'-Bis-(benzoylthio)-isobutyryl]-D,L-3-(naphth-2-yl)-alanin, $R_f$ = 0,37 in System II.
D,L-2-[3,3'-Bis-(benzoylthio)-isobutyramido]-4-phenylbuttersäure, Fp. 135 bis 136°C (Diethylether/Cyclohexan), $R_f$ = 0,38 in System II.
N-[3,3'-Bis-(benzoylthio)-isobutyryl]-tryptophan, Fp. 135 bis 139°C (Diethylether/Petrolether), $R_f$ = 0,35 in System II.
N-[3,3'-Bis-(benzoylthio)-isobutyryl]-D-tryptophan, Fp. 137 bis 140°C (Diethylether/Petrolether), $R_f$ = 0,35 in System II.
N-[3,3'-Bis-(benzoylthio)-isobutyryl]-N-methyl-tryptophan, $R_f$ = 0,37 in System II.
N-[3,3'-Bis-(benzoylthio)-isobutyryl]-alanyl-prolin, Fp. ab 60°C (Methylenchlorid/Petrolether), $R_f$ = 0,39 in System I.
N-[3,3'-Bis-(benzoylthio)-isobutyryl]-prolyl-phenylalanin, Fp. 134 bis 135°C (Diethylether), $R_f$ = 0,47 in System I.
N-[3,3'-Bis-(benzoylthio)-isobutyryl]-prolyl-prolin, $R_f$ = 0,23 in System I.
N-[3,3'-Bis-(benzoylthio)-isobutyryl]-phenylalanyl-alanin, $R_f$ = 0,62 in System I.
N-[3,3'-Bis-(benzoylthio)-isobutyryl]-valyl-prolin, $R_f$ = 0,54 in System I.
N-[3,3'-Bis-(benzoylthio)-isobutyryl]-valyl-tryptophan, $R_f$ = 0,58 in System I.

**Patentansprüche**

1. Bis-(benzoylthio)-carbonsäuren der Formel I

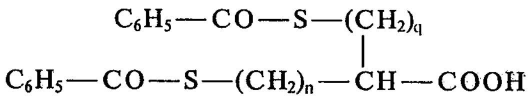

(I)

in der
n und q gleich oder verschieden sind und die Zahlen 0 oder 1 bedeuten.

2. Verfahren zur Herstellung von Verbindungen der Formel I gemäß Anspruch 1, dadurch gekennzeichnet, daß man

a) Verbindungen der Formel II

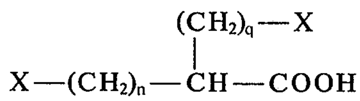

(II)

worin n und q die angegebene Bedeutung haben und X ein Halogenatom darstellt, mit Thiobenzoesäure oder

b) — falls n und q beide die Zahl 1 bedeuten — eine Verbindung der Formel III

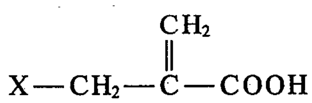

(III)

in der R und X die angegebene Bedeutung haben, mit Thiobenzoesäure umsetzt

und die so erhaltenen Verbindungen — falls n≠q — gegebenenfalls in ihre optischen Antipoden trennt.

3. Verwendung der Verbindungen der Formel I gemäß Anspruch 1 zur Herstellung von Benzoylthio-Verbindungen der Formel IV

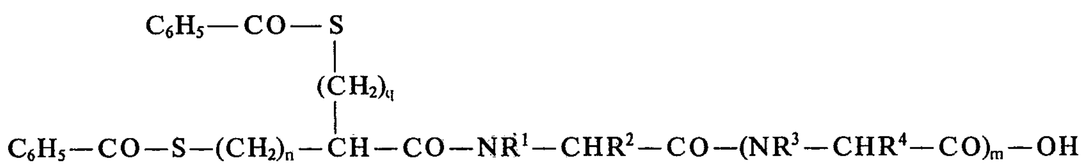

(IV)

worin
$R^1$ und $R^3$ gleich oder verschieden sind und Wasserstoffatome oder $C_{1-4}$-Alkylgruppen darstellen,
$R^2$ und $R^4$ gleich oder verschieden sind und Wasserstoff, einen gegebenenfalls durch eine Hydroxy-, Mercapto-, $C_{1-4}$-Alkylthio-, Carbonsäureester-, Carbonamido- oder Acylaminogruppe substituierten $C_{1-6}$-Alkylrest oder einen gegebenenfalls im Arylrest durch Hydroxy-, $C_{1-4}$-Alkoxy-, Arylalkoxygruppen oder Halogenatome substituierten Aryl-, Heteroaryl-, Arylalkylen- oder Heteroarylalkylenrest bedeuten, wobei $R^1$ und $R^2$ und/oder $R^3$ und $R^4$ zusammen auch die Reste $-(CH_2)_p-$ (p = 2, 3, 4), $-CH_2-S-CH_2-$ oder $-CH_2-C_6H_4-CH_2-$ (ortho) sein können und
q, m und n gleich oder verschieden sind und die Zahlen 0 oder 1 bedeuten,

sowie deren Salze mit physiologisch verträglichen Basen.

**Claims**

1. A bis(benzoylthio)carboxylic acid of the formula I

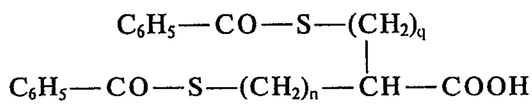

(I)

where
n and q are identical or different and are each 0 or 1.

2. A process for the preparation of a compound of the formula I as claimed in claim 1, wherein

a) a compound of the formula II

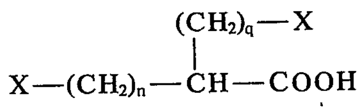

(II)

where n and q have the above meanings and X is halogen, is reacted with thiobenzoic acid, or

b) if n and q are each 1, a compound of the formula III

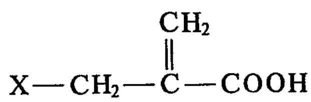

(III)

where R and X have the above meaning, is reacted with thiobenzoic acid,

and, if n and q are different, the resulting compound is, if desired, resolved into its optical antipodes.

3. Use of a compound of the formula I as claimed in claim 1 for the preparation of a benzoylthio compound of the formula IV

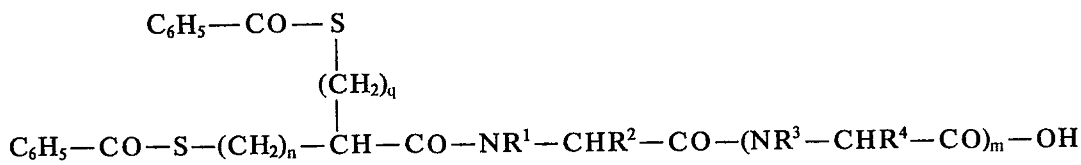

(IV)

where
$R^1$ and $R^3$ are identical or different and are each hydrogen or $C_1-C_4$-alkyl,
$R^2$ and $R^4$ are identical or different and are each hydrogen or $C_1-C_6$-alkyl which is unsubstituted or substituted by hydroxyl, mercapto, $C_1-C_4$-alkylthio, a carboxylic ester group, carboxamido or acylamino, or are each an aryl, hetaryl, arylalkylene or hetarylalkylene radical which is unsubstituted or substituted in the aryl moiety by hydroxyl, $C_1-C_4$-alkoxy, arylalkoxy or halogen, $R^1$ and $R^2$ and/or $R^3$ and $R^4$ together may furthermore be $-(CH_2)_p-$ ($p = 2, 3$ or 4), $-CH_2-S-CH_2-$ or $-CH_2-C_6H_4-CH_2-$ (ortho), and
q, m and n are identical or different and are each 0 or 1,

and its salts with physiologically tolerated bases.

**Revendications**

1. Acides bis-(benzoylthio)-carboxyliques de formule I

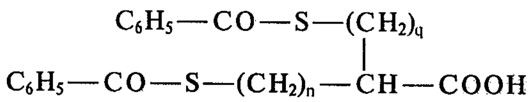

(I)

dans laquelle
n et q sont identiques ou différents et représentent les chiffres 0 ou 1.

2. Procédé de préparation de composés de formule I selon la revendication 1, caractérisé par le fait que l'on fait réagir:

a) des composés de formule II

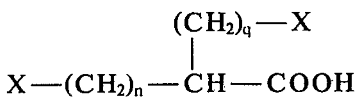

(II)

où n et q ont la signification sus-indiquée et X représente un atome d'halogène, avec un acide thiobenzoïque ou

b) — si n et q représentent tous deux le chiffre 1 — un composé de formule III

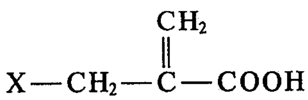

(III)

dans laquelle R et X ont la signification sus-indiquée, avec un acide thiobenzoïque et l'on sépare les composés ainsi obtenus, lorsque $n \neq q$, éventuellement, en leurs antipodes optiques.

3. Utilisation des composés de formule I selon la revendication 1 pour la préparation de composés benzoylthio de formule IV

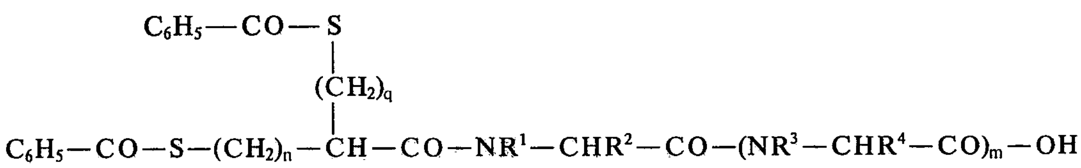

(IV)

dans laquelle
$R^1$ et $R^3$ sont identiques ou différents et représentent un atome d'hydrogène ou des groupes alkyle en $C_{1-4}$,
$R^2$ et $R^4$ sont identiques ou différents et représentent hydrogène, un reste alkyle en $C_{1-6}$, éventuellement substitué par un groupe hydroxy, mercapto, alkylthio en $C_{1-4}$, ester carboxylique, carbonamido ou acylamino, ou un reste aryle, hétéroaryle, arylalkyle ou hétéroalkyle éventuellement substitué dans le reste aryle par des groupes hydroxy, alcoxy en $C_{1-4}$, arylalcoxy ou des atomes d'halogène, $R^1$ et $R^2$ et/ou $R^3$ et $R^4$ pouvant être ensemble aussi les restes $-(CH_2)_p-$ ($p = 2, 3, 4$), $-CH_2-S-CH_2-$ ou $-CH_2-C_6H_4-CH_2-$ (ortho) et
q, m et n sont identiques ou différents et représentent les chiffres 0 ou 1,

ainsi que leurs sels de bases compatibles physiologiquement.